(19) Europäisches Patentamt
European Patent Office
Office européen des brevets

(11) Veröffentlichungsnummer: **0 010 585**
**B1**

# (12) EUROPÄISCHE PATENTSCHRIFT

(45) Veröffentlichungstag der Patentschrift: **13.05.81**

(51) Int. Cl.³: **C 07 C 125/065**

(21) Anmeldenummer: **79103156.0**

(22) Anmeldetag: **27.08.79**

## (54) Verfahren zur Herstellung von Urethanen.

(30) Priorität: **06.09.78 DE 2838754**

(43) Veröffentlichungstag der Anmeldung: **14.05.80 Patentblatt 80/10**

(45) Bekanntmachung des Hinweises auf die Patenterteilung: **13.05.81 Patentblatt 81/19**

(84) Benannte Vertragsstaaten: **BE DE FR GB IT NL**

(56) Entgegenhaltungen:
**DE-A-2 343 826**
**DE-A1-2 623 694**
**DE-B2-2 555 557**
**EP-A1-0 000 563**
**EP-A1-0 003 989**
**US-A-4 134 880**

(73) Patentinhaber: **BAYER AG, Zentralbereich Patente, Marken und Lizenzen, D-5090 Leverkusen 1, Bayerwerk (DE)**

(72) Erfinder: **Scholl, Hans-Joachim, Dr., Rudolf-Sohm-Strasse 28, D-5000 Koeln 60 (DE)**
Erfinder: **Zenner, Armin, Dr., Goethestrasse 65, D-4047 Dormagen (DE)**

**EP 0 010 585 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# Verfahren zur Herstellung von Urethanen

Die vorliegende Erfindung betrifft ein verbessertes Verfahren zur Herstellung von Urethanen durch Umsetzung von aromatischen Nitroverbindungen mit Alkoholen und Kohlenmonoxid in Gegenwart von Schwefel und/oder Selen und/oder Verbindungen dieser Elemente enthaltenden Katalysatorensystemen. Aus den genannten Ausgangsmaterialien zugängliche Urethane wurden bisher im allgemeinen durch Umsetzung eines aromatischen Isocyanats mit einem Alkohol gebildet, wobei das Isocyanat seinerseits durch Umsetzung von Phosgen mit dem entsprechenden primären Amin gewonnen wird, welches im allgemeinen wiederum durch Reduktion der entsprechenden Nitroverbindung erhalten worden war Dieses herkömmliche Verfahren weist jedoch verschiedene Nachteile auf, nicht zuletzt aufgrund der Toxizität und der korrosiven Natur von Phosgen und der Bildung von Chlorwasserstoff als Nebenprodukt. Ausserdem ist es bekannt, dass bestimmte aromatische Amine schädliche biologische Eigenschaften aufweisen und einige auch dazu neigen, bei der Lagerung durch Luft oxydiert zu werden.

Es mangelt daher auch nicht an Versuchen, den Umweg über das hochtoxische Phosgen zu vermeiden und die Urethane direkt aus den entsprechenden Nitroverbindungen und den entsprechenden Alkoholen sowie Kohlenmonoxid herzustellen. Die Verfahren der US-PS 3993685 bzw. der DE-OS 2603574 bedienten sich hierbei Katalysatorsystemen auf Basis von Metallen der Platin-Gruppe. Da bei diesen Verfahren merkliche Verluste der sehr kostspieligen Katalysatoren nicht vermieden werden können, fanden diese Verfahren bislang keinen Eingang in die grosstechnische Praxis.

Beim Verfahren der DE-OS 2343826 wird als katalytisch wirkendes System eine Kombination aus Selen oder Schwefel bzw. Verbindungen dieser Elemente mit sehr grossen Mengen einer Base vorgeschlagen. Als Basen kommen z. B. Triäthylamin und Pyridin in Frage. Um aber die Reaktion in Anwesenheit dieser Amine zufriedenstellend einzuleiten, erscheint es erforderlich, die tertiären Amine in einer ziemlich grossen Menge im Hinblick auf die Ausgangsnitroverbindungen zu verwenden. In der Tat wird, wenn Dinitrotoluol als Nitroverbindung verwendet wird, das tertiäre Amin in einer Menge verwendet, die gleich oder grösser ist als diejenige des Dinitrotoluols. Die Verwendung derart grosser Mengen an tertiärem Amin bringt zahlreiche Probleme wirtschaftlicher Art und auch hinsichtlich der Rückgewinnungsverfahren mit sich. Weiterhin führt dieses Verfahren zur Bildung von Nebenprodukten, z. B. Aminoverbindungen und Harnstoffen, wenn messbare Anteile an Wasser, z. B. als Hydrate, aber auch in freier Form vorliegen.

Auch das Verfahren der DE-OS 2343826 ist daher für eine grosstechnische Anwendung weitgehend ungeeignet.

Die wegen der genannten Bildung von Nebenprodukten herabgesetzte Ausbeute an den Urethanen lässt sich beim Verfahren der DE-OS 2614101 durch ein Katalysator-System vermeiden, welches aus elementarem Selen bzw. einer Selenverbindung und einem Promotor, bestehend z. B. aus einem bicyclischen Amidin und einer Carbonsäure zusammengesetzt ist. Zwar sind beim Verfahren der DE-OS 2614101 die Ausbeuten an Urethanen im Vergleich zum Verfahren der DT-OS 2343826 erhöht, jedoch entstehen auch hierbei störende Mengen an Nebenprodukten, welche insbesondere Hydrolyse- und Folgeprodukte des gebildeten Urethans darstellen.

Das Verfahren der DE-OS 2623694 muss insofern als eine Weiterentwicklung des Verfahrens der DE-OS 2614101 betrachtet werden, als die Bildung der Nebenprodukte aus den Urethanen durch Mitverwendung von diesen Nebenprodukten entsprechenden aromatischen Aminoverbindungen bzw. aromatischen Harnstoffverbindungen zurückgedrängt wird. Obwohl mit dieser Massnahme eine Verbesserung des Verfahrens gemäss DE-OS 2614101 möglich ist, ist auch das Verfahren der DE-OS 2623694 noch mit schwerwiegenden Mängeln behaftet. Insbesondere ist bei diesem Verfahren die Verwendung von ungewöhnlich hohen Mengen an Selen bzw. Selenverbindungen erforderlich, was zu erheblichen Verlusten dieses Katalysators führt. Darüber hinaus handelt es sich bei Selen bzw. den einzusetzenden Selenverbindungen um toxikologisch bedenkliche Substanzen, die darüber hinaus dem hergestellten Urethan einen unangenehmen Geruch verleihen.

Es war daher Aufgabe der vorliegenden Erfindung, ein verbessertes Verfahren zur Herstellung von Urethanen aus aromatischen Nitroverbindungen, Alkoholen und Kohlenmonoxid zur Verfügung zu stellen, bei welchem entweder ganz ohne Selen bzw. Selenverbindungen gearbeitet wird oder bei welchem die Menge des Selens bzw. der Selenverbindung ganz wesentlich reduziert werden kann und dennoch eine möglichst quantitative Urethanbildung erfolgt.

Diese Aufgabe konnte überraschenderweise durch das nachstehend näher erläuterte erfindungsgemässe Verfahren gelöst werden, bei welchem die geschilderten Reinigungs-, Toxikologie- und Abtrennungsprobleme weitgehend entfallen.

Gegenstand der vorliegenden Erfindung ist ein Verfahren zur Herstellung von Urethanen durch Umsetzung von aromatischen Nitroverbindungen mit aliphatischen, cycloaliphatischen oder araliphatischen Alkoholen und Kohlenmonoxid in Gegenwart von

a) Schwefel und/oder Selen und/oder Verbindungen dieser Elemente,

b) aromatische Aminoverbindungen und/oder aromatische Harnstoffverbindungen, sowie

c) tertiäre organische Amine und/oder Alkalisalze

schwacher Säuren aufweisenden Katalysatorensystemen, sowie

d) Oxidationsmittel ausgewählt aus der Gruppe bestehend aus Sauerstoff, oxidierend wirkenden, chemisch gebundenen Sauerstoff enthaltenden organischen Verbindungen und oxidierend wirkenden, chemisch gebundenen Sauerstoff enthaltenden anorganischen Verbindungen von Metallen der 1., 2., 5.–8. Nebengruppe des Periodensystems der Elemente,

dadurch gekennzeichnet, dass man Katalysatorensysteme verwendet, welche als zusätzliche Komponente

e) Ammoniak und/oder aliphatische, araliphatische, cycloaliphatische oder heterocyclische Amine mit mindestens einem an Aminstickstoff gebundenen Wasserstoffatom enthalten.

Die nicht vorveröffentlichte, ältere europäische Patentanmeldung 39 89 hat ein ähnliches Verfahren zum Gegenstand. Dieses Verfahren der älteren Anmeldung unterscheidet sich vom vorliegenden erfindungsgemässen Verfahren jedoch durch den Umstand, dass auf die Mitverwendung der obgenannten Komponente e) verzichtet wird.

Ausgangsverbindungen für das erfindungsgemässe Verfahren sind

1. aromatische Nitroverbindungen, wie z. B. Nitrobenzol, 4-Nitrochlorbenzol, 3,4-Dichlornitrobenzol, 1,3-Dinitrobenzol, o-Nitrotoluol, m-Nitrotoluol, p-Nitrotoluol, 2,4-Dinitrotoluol, 2,6-Dinitrotoluol, Nitronaphtaline, Nitroanthracene, Nitrobiphenylene u. dgl. Im allgemeinen weisen die erfindungsgemäss geeigneten Nitroverbindungen ein Molekulargewicht von 123–300, 1–3 aromatische Kerne, sowie 1–3 an aromatische Kerne gebundene Nitrogruppen, sowie gegebenenfalls weitere unter den Reaktionsbedingungen des erfindungsgemässen Verfahrens inerte Substituenten auf. Zu den bevorzugten Nitroverbindungen für das erfindungsgemässe Verfahren gehören Nitrobenzol, sowie die genannten Dinitrotoluole. Beliebige Gemische der genannten Nitroverbindungen können selbstverständlich ebenfalls eingesetzt werden.

2. Aliphatische, cycloaliphatische oder araliphatische Alkohole, d. h. vorzugsweise beliebige, unter den Reaktionsbedingungen ansonsten inerte organische Verbindungen mit mindestens einer aliphatischen, cycloaliphatisch oder araliphatisch gebundenen Hydroxylgruppe des Molekulargewichtsbereichs 32–300. Beispiele geeigneter Alkohole sind primäre, sekundäre oder tertiäre Alkohole wie z. B. Methanol, Äthanol, n-Propanol, iso-Propanol, die verschiedenen isomeren Butanole, Cyclohexylalkohol, Benzylalkohol, Hexylalkohol, Laurylalkohol u. dgl. Vorzugsweise werden beim erfindungsgemässen Verfahren einwertige Alkohole, besonders bevorzugt Äthanol und Methanol, eingesetzt.

3. Gasförmiges Kohlenmonoxid.

Beim erfindungsgemässen Verfahren werden Katalysatoren-Systeme eingesetzt, welche a) Schwefel und/oder Selen und/oder Verbindungen dieser Elemente, b) aromatische Aminoverbindungen und/oder aromatische Harnstoffverbindungen, c) mindestens ein tertiäres organisches Amin und/oder mindestens ein Alkalisalz einer schwachen Säure, d) bestimmte, nachstehend näher beschriebene Oxidationsmittel und e) Ammoniak und/oder mindestens ein aliphatisches, araliphatisches, cycloaliphatisches oder heterocyclisches Amin mit mindestens einem an Aminstickstoff gebundenem Wasserstoffatom aufweisen.

Geeignete Katalysatoren-Komponenten a) sind beispielsweise

aa) elementarer Schwefel in beliebiger Form, anorganische oder organische Verbindungen vorzugsweise des zweiwertigen Schwefels wie z. B. Carbonylsulfid (COS) Schwefelwasserstoff, Alkalisulfide wie z. B. Natriumsulfid, Dimethylsulfid, Diäthylsulfid, Thiopen oder Thioharnstoff. Bevorzugt werden elementarer Schwefel, Carbonylsulfid oder solche Schwefelverbindungen die unter den Reaktionsbedingungen des erfindungsgemässen Verfahrens in situ Carbonylsulfid bilden, eingesetzt. Ferner geeignet sind

ab) Selen in beliebiger Form, vorzugsweise metallisches Selen, oder anorganische Selenverbindungen wie z. B. Selendioxid oder Carbonylselenid (COSe). Prinzipiell denkbar ist auch die Verwendung von organischen Selenverbindungen, wie z. B. Dimethylselenid, Diphenylselenid u. dgl. Unter den unter ab) genannten Cokatalysatoren ist elementares Selen besonders bevorzugt.

Schwefel ist die bevorzugte Katalysatoren-Komponente a).

Bei der Katalysatoren-Komponente b) handelt es sich um beliebige aromatische gebundene primäre Aminogruppen und/oder aromatisch gebundene Harnstoffgruppen aufweisende organische Verbindungen, die neben den genannten Gruppen, insbesondere auch noch Nitrogruppen und Urethangruppen aufweisen können. Im allgemeinen handelt es sich bei der Komponente b) der erfindungsgemäss einzusetzenden Katalysatoren-Systeme um Verbindungen bzw. Gemische von Verbindungen, welche den Formeln

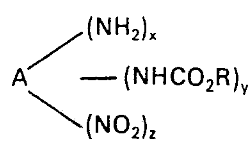

und/oder

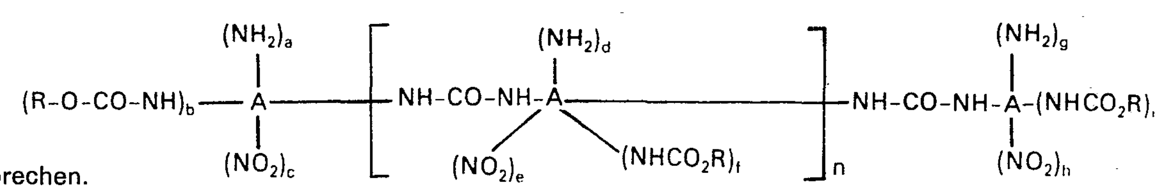

entsprechen.

In diesen Formeln bedeuten

x 1 oder 2,

y 0 oder 1,

z 0 oder 1, wobei die Summe x + y + z vorzugsweise 1 oder 2 beträgt,

a,b,c,d,e,f,g,h und i jeweils 0 oder 1, wobei die Summe a + b + c gleich der Summe g + h + i und 0, 1 oder 2 bedeutet, während die Summe d + e + f im Falle von a + b + c = 1 oder 2 einen um 1 verminderten Wert, d. h. 0 oder 1 darstellt, und im Fall von a + b + c = 0 ebenfalls für 0 steht,

n 0, 1, 2 oder 3, vorzugsweise jedoch 0 bedeutet,

A einen 1-, 2- oder 3-wertigen, vorzugsweise 1- oder 2-wertigen, gegebenenfalls $C_1$-$C_4$-Alkyl-substituierten aromatischen Kohlenwasserstoffrest, der im übrigen vorzugsweise dem aromatischen Kohlenwasserstoffrest der beim erfindungsgemässen Verfahren einzusetzenden aromatischen Nitroverbindung entspricht und

R einen aliphatischen, cycloaliphatischen oder araliphatischen Kohlenwasserstoffrest mit im allgemeinen bis zu 18 Kohlenstoffatomen, der im übrigen vorzugsweise dem Kohlenwasserstoffrest der beim erfindungsgemässen Verfahren einzusetzenden Alkoholkomponente entspricht.

Beispiele geeigneter Katalysatoren-Komponenten b) sind Anilin, o-, m- oder p-Toluidin, die isomeren Nitroaniline, die isomeren Diaminobenzole, N,N'-Diphenylharnstoff, N,N'-Bis(2-methyl-5-nitro-phenyl)-harnstoff, N,N'-Bis-(2-methyl-5-äthoxycarbonylamino-phenyl)-harnstoff, N,N'-Bis-(2-methyl-5-amino-phenyl)-harnstoff, 2-Amino-4-nitro-toluol, 4-Amino-2-nitrotoluol, 2-Amino-4-äthoxycarbonyl-amino-toluol, 4-Amino-2-äthoxycarbonylamino-toluol, 2,4-Diaminotoluol, N,N'-Bis-(3-nitro-4-methyl-phenyl)-harnstoff, N,N'-Bis-(2-methyl-5-nitrophenyl)-harnstoff, N,N'-Bis-(3-äthoxycarbonylamino-4-methylphenyl)-harnstoff, N,N'-Bis-(2-methyl-5-äthoxycarbonylaminophenyl)-harnstoff, N,N'-Bis-(3-amino-4-methylphenyl)-harnstoff, N,N'-Bis-(2-methyl-5-aminophenyl)-harnstoff, N-(3-Nitro-4-methylphenyl)-N'-(2-methyl-5-nitrophenyl)-harnstoff, N-(3-äthoxycarbonylamino-4-methylphenyl)-N'-(2-methyl-5-äthoxycarbonylamino)-harnstoff, N-(3-Amino-4-methylphenyl)-N'-(2-methyl-5-aminophenyl)-harnstoff, N-(3-Nitro-4-methylphenyl)-N'-(3-äthoxycarbonylamino-4-methylphenyl)-harnstoff, N-(3-Nitro-4-methylphenyl)-N'-(2-methyl-5-äthoxycarbonylaminophenyl)-harnstoff, N-(3-Nitro-4-methylphenyl)-N'-(3-amino-4-methyl-phenyl)-harnstoff, N-(3-Nitro-4-methylphenyl)-N'(2-methyl-5-aminophenyl)-harnstoff, N-(2-Methyl-5-nitrophenyl)-N'-(3-äthoxycarbonylamino-4-methylphenyl)-harnstoff, N-(2-Methyl-5-nitrophenyl)-N'-(2-methyl-5-äthoxycarbonylaminophenyl)-harnstoff, N-(2-Methyl-5-nitrophenyl)-N'-(3-amino-4-methylphenyl)-harnstoff, N-(2-Methyl-5-nitrophenyl)-N'-(2-methyl-5-aminophenyl)-harnstoff, N-(3-Äthoxycarbonylamino-4-methylphenyl)-N'-(2-methyl-5-aminophenyl)-harnstoff, N-(2-Methyl-5-äthoxycarbonylaminophenyl)-N'-(3-amino-4-methylphenyl)-harnstoff, N-(2-Methyl-5-äthoxycarbonylaminophenyl)-N'-(2-methyl-5-aminophenyl)-harnstoff, sowie beliebige Gemische der beispielhaft genannten Verbindungen. Wie bereits dargelegt, werden vorzugsweise solche Verbindungen b) eingesetzt, die bezüglich ihres aromatischen Restes der beim erfindungsgemässen Verfahren einzusetzenden aromatischen Nitroverbindung entsprechen. Bei Verwendung von Nitrobenzol wird demgemäss beispielsweise Anilin oder Diphenylharnstoff verwendet, während bei Verwendung von Nitrotoluol entweder ein Toluoylamin oder ein Ditoluolharnstoff zum Einsatz gelangt. Dementsprechend werden bei Verwendung zweiwertiger Nitroverbindungen beispielsweise von 2,4-Dinitrotoluol, die entsprechenden, zweifach substituierte Toluoylreste aufweisenden Verbindungen eingesetzt.

Höhere Homologe der beispielhaft aufgeführten Harnstoffe, d. h. mehrere Harnstoffeinheiten aufweisende Verbindungen können ebenfalls eingesetzt werden.

Bei der Katalysatoren-Komponente c) handelt es sich um tertiäre Aminogruppen aufweisende organische Basen wie z. B. tertiäre aliphatische Amine mit insgesamt 3 bis 20 Kohlenstoffatomen wie Trimethylamin, Triäthylamin, N,N-Dimethyl-octadecylamin oder Trihexylamin, heterocyclische tertiäre Amine wie z. B. Pyridin oder 2 tert. Aminogruppen aufweisende Amine wie z. B. Diazabicyclo[2. 2. 2]-octan (Triäthylendiamin), oder bicyclische Amidine der Formel

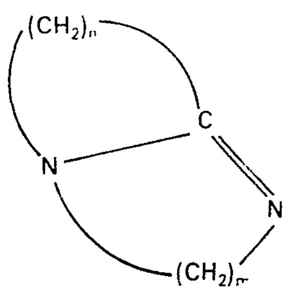

in welcher

n für eine ganze Zahl von 3 bis 5 und

m für eine ganze Zahl von 2 bis 4 stehen.

Neben oder anstelle der genannten tertiären Amine können als Katalysator-Komponente c) auch basisch wirkende Alkalisalze schwacher Säuren insbesondere Alkalicarboxylate wie Natriumacetat, Kaliumacetat, Natriumbenzoat oder Alkalisalze schwacher anorganischer Säuren wie z. B. Natriumborat oder Natriumcarbonat verwendet werden. Zu den bevorzugten Katalysator-Komponenten c) gehören 1,5-Diazabicyclo[4.3.0]-non-5-en, 1,8-Diazabicyclo[5.4.0]-undecen-7 und Natrium- bzw. Kaliumacetat. Bevorzugt kommt weiterhin Triäthylendiamin, insbesondere in Kombination mit Salzen der Formel

MeX

zum Einsatz, wobei

Me für ein Alkalimetall-Kation und

X für ein Jodid-, Cyanat- oder Rhodanid-Anion stehen.

Bei Verwendung derartiger Kombinationen werden die letztgenannten Salze im allgemeinen in Mengen von 1 bis 40 Mol-% vorzugsweise von 4 bis 20 Mol-% bezogen auf die eingesetzte Nitroverbindung verwendet.

Bei den Oxidationsmitteln d) handelt es sich entweder um elementaren Sauerstoff bzw. ein Sauerstoff enthaltendes Gas wie z. B. Luft und/oder um oxidierend wirkende, chemisch gebundenen Sauerstoff aufweisende organische Verbindungen wie z. B. Chinone vorzugsweise 1,4-Benzochinon und/oder um oxidierend wirkende chemisch gebundenen Sauerstoff aufweisende anorganische Verbindungen von Metallen. Bei den letztgenannten Verbindungen handelt es sich insbesondere um die entsprechenden Oxide. Es kommen vorzugsweise die entsprechenden Metallverbindungen der Elemente der 1., 2. sowie 5.–8. Nebengruppen des Periodensystems in Betracht. Besonders bevorzugt werden jedoch die entsprechenden Verbindungen der Elemente der 5. und 6. Nebengruppe sowie die entsprechenden Verbindungen von Mangan, Eisen, Kobalt und Nickel eingesetzt. Beispiele geeigneter Oxidationsmittel sind Zinkoxid, Eisen-II-oxid, Eisen-III-oxid, aus diesen letztgenannten Eisenoxiden bestehende Mischoxide, Vanadium-V-oxid, Mangan-IV-oxid, Molybdän-VI-oxid, Nickel-II-oxid, Kobalt-II-oxid, Mischoxide des 3- bis 6-wertigen Chroms, sowie beliebige Gemische der beispielhaft genannten Oxide. Zu den besonders bevorzugten Oxidationsmitteln gehört Eisen-III-oxid. Ganz besonders bevorzugt sind Eisen, Vanadium und/oder Molybdän enthaltende Mischoxide.

Bei der Katalysatoren-Komponente e) handelt es sich um Ammoniak und/oder beliebige aliphatische, araliphatische, cycloaliphatische oder heterocyclische Amine mit mindestens einem an Aminstickstoff gebundenen Wasserstoffatom. Bevorzugt werden als Katalysatoren-Komponente e) sekundäre Amine der Formel

$R_1NH-R_2$

eingesetzt, wobei
$R_1$ und $R_2$ für gleiche oder verschiedene Reste stehen und Alkylreste mit 1 bis 6 Kohlenstoffatomen, Cycloalkylreste mit 5 bis 6 Kohlenstoffatomen bedeuten, oder wobei $R_1$ und $R_2$ zusammen mit dem sekundären Aminstickstoffatom einen gegebenenfalls auch Sauerstoff als zweites Heteroatom enthaltenden, vorzugsweise 6-gliedrigen heterocyclischen Ring bilden.

Zu den bevorzugten Aminen gehören Dimethylamin, Diäthylamin, Dipropylamin, Dibutylamin, Methyl-hexyl-amin, Dihexylamin oder Morpholin. Dibutylamin oder Morpholin sind besonders bevorzugt.

Selbstverständlich sind als Katalysatoren-Komponente e) auch Verbindungen geeignet, die unter den erfindungsgemässen Verfahrensbedingungen Amine insbesondere der genannten Formel $R_1-NH-R_2$ freisetzen. Ein typisches Beispiel für eine derartige Substanzklasse sind Thioharnstoffe, die unter Hydrolysebedingungen in bekannter Reaktion zu den entsprechenden Aminen ausspalten (vgl. Frost Pearson Kinetics and Mechanism, John Wiley and Sons, New York (1961), Seite 314):

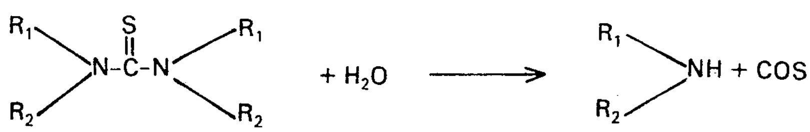

Derartige Verbindungen sind deswegen von besonderem Interesse, weil vorhandene, unerwünschte Restspuren von Wasser im Reaktionssystem weitgehend entfernt werden und zwar unter gleichzeitiger Ausbildung erfindungsgemäss einzusetzender Katalysatoren-Komponenten.

Bei der Durchführung des erfindungsgemässen Verfahrens kommen die Reaktionspartner im allgemeinen in solchen Mengen zum Einsatz, dass für jede Nitrogruppe der als Ausgangsmaterial eingesetzten aromatischen Nitroverbindung zwischen 1 bis 50 vorzugsweise zwischen 5 bis 30 Hydroxylgruppen der Alkoholkomponente vorliegen. Das Kohlenmonoxid wird im allgemeinen im Überschuss eingesetzt, da stets in Kohlenmonoxid-Atmosphäre gearbeitet wird; die gegebenenfalls den erfindungsgemässen Anteil Sauerstoff aufweist.

Die Katalysatoren-Komponente a), welche auch auf einen geeigneten Träger wie Kohlenstoff, Aluminiumoxid, Siliciumdioxid, Diatomeenerde, aktivierter Ton, Zeolith, Molekularsieben, Bariumsulfat, Kalziumcarbonat, Ionenaustauscherharzen und ähnlichen Materialien aufgebracht werden kann, wird im Falle der Verwendung von Schwefel oder Schwefelverbindungen im allgemeinen in einer solchen Menge eingesetzt, die 0,1 bis 40 Gew.-%, vorzugsweise 1 bis 15 Gew.-% Schwefel in freier oder gebundener Form, bezogen auf die als Ausgangsmaterial eingesetzte Nitroverbindung entspricht und im Falle der Verwendung von Selen oder von Selenverbindungen in einer Menge, die 0,001 bis 1 Gew.-%, vorzugsweise 0,01 bis 0,5 Gew.-% an freiem oder gebundenem Selen bezogen auf die Nitroverbindung entspricht.

Die Katalysatoren-Komponente b) liegt im Reaktionsgemisch im allgemeinen in einer Menge von 1 bis 40 Mol.-%, vorzugsweise 4 bis 25 Mol-% bezogen auf als Ausgangsmaterial eingesetzte Nitroverbindung vor. Die Katalysatoren-Komponente c) liegt im Reaktionsgemisch im allgemeinen in einer Menge von 1 bis 40, vorzugsweise 4 bis 25 Mol-%, bezogen auf als Ausgangsmaterial verwendete Nitroverbindung vor, wobei

sich diese Angaben auf die Gesamtmenge an basischen Verbindungen nicht jedoch auf die gegebenenfalls mitzuverwendenden Salze der Formel

MeX

beziehen.

Die Katalysatoren-Komponente d), d. h. das Oxidationsmittel wird bei Verwendung von Sauerstoff bzw. von Sauerstoff enthaltendem Gas im allgemeinen so gewählt, dass der Sauerstoffanteil 0,01 bis 6,0 Vol.-%, vorzugsweise 0,1 bis 2 Vol.-% bezogen auf eingesetztes Kohlenmonoxid beträgt. Aus Sicherheitsgründen sollten 6,0 Vol.-% nicht überschritten werden. Im Falle der Verwendung von oxidierend wirkenden Metallverbindungen werden diese im allgemeinen in Mengen von 0,1 bis 100 Gew.-%, vorzugsweise 5 bis 40 Gew.-%, bezogen auf die eingesetzte Nitroverbindung, verwendet.

Die Katalysatoren-Komponente e) liegt im Reaktionsgemisch im allgemeinen in einer Menge von 0,01 bis 20 Mol.-%, vorzugsweise 0,1 bis 15 Mol-%, bezogen auf als Ausgangsmaterial eingesetzte Nitroverbindung vor.

Das erfindungsgemässe Verfahren kann in Abwesenheit eines Lösungsmittels erfolgen, da der Alkohol als Lösungsmittel dient, jedoch kann auch ein Lösungsmittel verwendet werden. Beispiele für solche Lösungsmittel umfassen aromatische Lösungsmittel, wie Benzol, Toluol, Xylol usw., Nitrile, wie Acetonitril, Benzonitril, usw., Sulfone, wie Sulfolan, aliphatische halogenierte Kohlenwasserstoffe, wie 1,1,2-Trichlor-1,2,2-trifluoräthan, aromatische halogenierte Kohlenwasserstoffe, wie Monochlorbenzol, Dichlorbenzol, Trichlorbenzol usw., Ketone, Ester und andere Lösungsmittel, wie Tetrahydrofuran, 1,4-Dioxan, 1,2-Dimethoxyäthan und ähnliches.

Die Reihenfolge der Zugabe der Ausgangsmaterialien und des Katalysatorensystems ist nicht beschränkt und kann wahlfrei je nach der Art der verwendeten Apparatur verändert werden. Beispielsweise wird eine Ausgangsmischung aus Alkohol, organische Nitroverbindung und den Katalysatoren-Komponenten a) – e) in einem geeigneten druckbeständigen Reaktor, wie ein Autoklav, eingeführt, worauf weiter Kohlenmonoxid unter Druck eingebracht wird, wonach unter Erwärmung gerührt wird, bis die Urethanbildungsreaktion beendet ist. Kohlenmonoxid und gegebenenfalls das Oxidationsmittel kann in den Reaktor entweder halbkontinuierlich oder kontinuierlich eingeleitet werden, während das beim Fortschreiten der Reaktion gebildete Kohlendioxid abgetrennt wird. Die Reaktion kann sowohl ansatzweise als auch halbkontinuierlich oder kontinuierlich durchgeführt werden. Das nach Beendigung der Reaktion im Überschuss anwesende Kohlenmonoxid kann durch Rezirkulation erneut verwendet werden. Die Reaktionstemperatur wird im allgemeinen im Bereich von 80 bis 220°C, gehalten. Obwohl die Reaktion bei höheren Reaktionstemperaturen schneller abläuft, besteht bei Temperaturen oberhalb 220°C die Neigung zu einer thermischen Zersetzung, wodurch die Ausbeute an Urethanprodukt verringert wird. Der Reaktionsdruck, d. h. der anfängliche Kohlenmonoxiddruck vor Beginn des Aufheizens, liegt im allgemeinen im Bereich von 10 bis 300 bar, vorzugsweise 20 bis 150 bar. Die Reaktionszeit hängt von der Natur und Art der verwendeten Nitroverbindung, der Reaktionstemperatur, dem Reaktionsdruck, der Art und Menge des Katalysators und der Art der Apparatur ab, jedoch liegt sie im allgemeinen im Bereich von 5 Minuten bis 6 Stunden. Nach Beendigung der Reaktion wird die Reaktionsmischung der Abkühlung überlassen bzw. abgekühlt. Nach dem Ablassen des eingefüllten Gases wird die Reaktionsmischung einer bekannten Abtrennung, wie Filtration, Destillation oder eine andere geeignete Methode, unterworfen, um das gebildete Urethan abzutrennen.

Die nach Abtrennung des Urethans zurückbleibenden Reaktionsanteile enthalten das Katalysatorsystem sowie nicht abgetrennte Urethanrestanteile. Die Wiederverwendung dieser Rückstände ist insbesondere bei kontinuierlicher Verfahrensweise von Vorteil.

Bei der Durchführung des erfindungsgemässen Verfahrens sollte auf Wasserausschluss geachtet werden, da trotz des Zusatzes der Katalysatoren-Komponenten b) eine teilweise hydrolytische Spaltung der erfindungsgemässen Verfahrensprodukte bei Anwesenheit von Wasser nicht ausgeschlossen werden kann.

Der erfindungswesentliche Punkt ist beim erfindungsgemässen Verfahren in erster Linie in der gleichzeitigen Mitverwendung der Katalysatoren-Komponenten d) und e) zu sehen, die in Kombination mit den Katalysatoren-Komponenten b) und c) selbst dann eine ausgezeichnete katalytische Wirksamkeit ermöglichen, wenn als Katalysatoren-Komponente a) Selen bzw. Selenverbindungen in, im Vergleich zu dem Verfahren des Standes der Technik, ganz drastisch reduzierten Mengen eingesetzt wird, bzw. wenn auf die Verwendung von Selen bzw. von Selenverbindung überhaupt verzichtet und an ihrer Stelle Schwefel bzw. Schwefelverbindungen zum Einsatz gelangen. Im Augenblick steht für diese überraschende Wirkung des erfindungsgemässen Katalysatoren-Systems keine plausible Erklärung zur Verfügung.

Die erfindungsgemässen Verfahrensprodukte stellen wertvolle Zwischenprodukte zur Herstellung von Schädlingsbekämpfungsmitteln oder zur Herstellung von Polyurethanen dar. Insbesondere eignen sich die erfindungsgemässen Verfahrensprodukte als Ausgangsmaterialien zur Herstellung der entsprechenden Isocyanate bzw. Polyisocyanate durch an sich bekannte Abspaltung der Alkoholkomponente.

Die nachfolgenden Beispiele veranschaulichen die Erfindung ohne sie jedoch einzuschränken. In den Beispielen wurden alle Reaktionen in einem mit einem Rührer versehenen Autoklaven aus rostfreiem Stahl (V 4A) durchgeführt. Die in den Beispielen angegebenen Ausbeuten wurden jeweils aus den Ergebnissen der Gaschromatographie und Flüssigkeitschromatographie errechnet.

Beispiel 1

20,8 g Nitrobenzol, 2 g Schwefel, 3,21 g Anilin, 2,36 g Kaliumacetat, 3 g eines Metalloxidgemisches aus Eisen-III-oxid und Vanadinpentoxid im Gewichtsverhältnis 1:1, 2,6 g Di-n-butylamin und 169 g abs. Äthanol wurden in einen 0,7 l Autoklaven eingebracht. Der Autoklav wurde mit Stickstoff und nachfolgend mit Kohlenmonoxid gespült. Anschliessend wurde Kohlenmonoxid in den Autoklaven unter Druck eingeleitet, bis der Ausgangsdruck 100 bar erreichte. Unter Rühren wurde auf 170°C aufgeheizt und 2 Stunden bei 170°C nachgerührt. Die gaschromatographische Analyse ergab eine quantitative Umwandlung des Nitrobenzols. Im Filtrat befanden sich 20,5 g Äthyl-N-phenylcarbamat und 4,5 g Anilin.

Im folgenden ist beispielhaft die Aufarbeitung des Ansatzes aus Beispiel 1 zur Isolierung des Äthyl-N-phenylcarbamats beschrieben:

Feste Bestandteile wurden abfiltriert und die Lösung zur Entfernung des Äthanols destilliert. Der Rückstand wurde in 80 g Toluol aufgenommen, der Toluol-Extrakt filtriert und das Filtrat mit Wasser ausgeschüttelt. Die organische Phase wurde der Destillation unterworfen. Nach Abtrennung des Toluols und Restmengen des Anilins gingen 21,2 g einer rasch kristallin werdenden Substanz bei 88 bis 94°C/0,2 bis 0,3 mbar über, die frei von anderen destillierbaren Verunreinigungen war und nach gaschromatographischer Analyse Äthyl-N-phenylcarbamat von 96-%iger Reinheit war.

Vergleichsbeispiele 1a

Beispiel 1 wurde ohne Di-n-butylamin wiederholt. Das Nitrobenzol war zu 43% umgesetzt. Das Filtrat enthielt 6,6 g Äthyl-N-phenylcarbamat und 3,7 g Anilin.

Vergleichsbeispiel 1b

Beispiel 1 wurde ohne Zusatz des Metalloxidgemisches wiederholt. Das Nitrobenzol war zu 70% umgesetzt. Das Filtrat enthielt 9,6 g Äthyl-N-phenylcarbamat und 3,8 g Anilin.

Beispiel 2

Beispiel 1 wurde mit 1,29 g Di-n-butylamin bei 160°C/2 Stunden wiederholt. Das Nitrobenzol war quantitativ umgewandelt. Das Filtrat enthielt 22,3 g Äthyl-N-phenyl-carbamat und 3,4 g Anilin.

Beispiel 3

Beispiel 2 wurde mit 0,87 g Morpholin anstatt Di-n-butylamin wiederholt. Das Nitrobenzol war quantitativ umgewandelt. Das Filtrat enthielt 22,2 g Äthyl-N-phenylcarbamat und 3,4 g Anilin.

Vergleichsbeispiel 3a

Beispiel 3 wurde ohne Zusatz des Metalloxidgemisches wiederholt. Das Nitrobenzol war zu 27,4% umgewandelt. Das Filtrat enthielt 2,2 g Äthyl-N-phenylcarbamat und 4 g Anilin.

Beispiel 4

Beispiel 3 wurde bei 180°C/1 Stunde wiederholt. Das Nitrobenzol war quantitativ umgewandelt. Das Filtrat enthielt 22,9 g Äthyl-N-phenylcarbamat und 3,9 g Anilin.

Beispiel 5

Beispiel 2 wurde mit 1,2 g Tetramethylthioharnstoff anstatt Di-n-butylamin wiederholt. Das Nitrobenzol war quantitativ umgewandelt. Im Filtrat befanden sich 24,6 g Äthyl-N-phenyl-carbamat und 2,7 g Anilin. Die Aufarbeitung dieses Ansatzes entsprechend Beispiel 1 erbrachte 24,4 g Äthyl-N-phenylcarbamat von 97-%iger Reinheit.

Beispiel 6

Beispiel 5 wurde mit 0,5 g Schwefel bei 170°C/1 Stunde wiederholt. Das Nitrobenzol war quantitativ umgewandelt. Im Filtrat befanden sich 21,5 g Äthyl-N-phenylcarbamat und 4,4 g Anilin.

Beispiel 7

51 g Nitrobenzol, 4 g Schwefel, 4 g Anilin, 2 g Kaliumacetat, 4 g eines Metalloxidgemisches gemäss Beispiel 1, 1,28 g Di-n-butylamin und 300 g abs. Äthanol wurden in einen 1,3 l Autoklaven eingebracht. Der Autoklav wurde mit Stickstoff und nachfolgend mit Kohlenmonoxid gespült. Anschliessend wurde Kohlenmonoxid in den Autoklaven unter Druck eingeleitet, bis der Ausgangsdruck 100 bar erreichte. Unter Rühren wurde auf 180°C aufgeheizt und 1 Stunde bei 180°C nachgerührt. Die gaschromatographische Analyse ergab eine quantitative Umwandlung des Nitrobenzols. Im Filtrat befanden sich 53,9 g Äthyl-N-phenylcarbamat und 8,2 g Anilin.

Beispiel 8

20,8 g Nitrobenzol, 2 g Schwefel, 3,21 g Anilin, 2,7 g Triäthylendiamin, 2,32 g Kaliumrhodanid, 3 g eines Metalloxidgemisches aus Eisen-III-oxid und Vanadinpentoxid im Gewichtsverhältnis 11:1, 0,87 g Morpholin und 169 g abs. Äthanol werden, wie in Beispiel 1 beschrieben, bei 180°C/1 Stunde umgesetzt. Das Nitrobenzol war quantitativ umgewandelt. Im Filtrat befanden sich 23,6 g Äthyl-N-phenylcarbamat und 4,1 g Anilin.

Vergleichsbeispiel 8a

Beispiel 8 wurde ohne Kaliumrhodanid wiederholt. Das Nitrobenzol war zu 45,7% umgesetzt. Im Filtrat befanden sich 6,6 g Äthyl-N-phenylcarbamat und 3,3 g Anilin.

Beispiel 9

20,8 g Nitrobenzol, 3 g COS, 3,21 g Anilin, 2,7 g Triäthylendiamin, 2,32 g Kaliumrhodanid, 3 g eines Metalloxidgemisches gemäss Beispiel 8, 0,64 g Di-n-butylamin und 169 g abs. Äthanol wurden, wie in Beispiel 1 beschrieben, bei 170°C/2 Stunden umgesetzt. Das Nitrobenzol war zu 91,8% umgesetzt. Im Filtrat befanden sich 21,8 g Äthyl-N-phenylcarbamat und 3,5 g Anilin.

Beispiel 10

51,0 g Nitrobenzol, 2 g Schwefel, 0,02 g Selen, 4 g Anilin, 2 g Kaliumacetat, 4 g Metalloxidgemisch gemäss Beispiel 1, 1,28 g Di-n-butylamin und 300 g abs. Äthanol wurden gemäss Beispiel 7 umgesetzt. Die gaschromatographische Analyse ergab eine quantitative Umwandlung des Nitrobenzols. Im Filtrat befanden sich 59,3 g Äthyl-N-phenylcarbamat und 7,3 g Anilin. Die Aufarbeitung dieses Ansatzes entsprechend Beispiel 1 erbrachte 58,1 g Äthyl-N-phenylcarbamat von 98,5-%iger Reinheit.

Beispiel 11

20,8 g Nitrobenzol, 2 g Schwefel, 3,21 g Anilin, 2,36 g Kaliumacetat, 3 g eines Metalloxidgemischs aus Eisen-III-oxid und Vanadinpentoxid im Gewichtsverhältnis 11:1, 0,87 g Morpholin und 130 g abs. Methanol wurden, wie in Beispiel 8 beschriebenen, umgesetzt. Das Nitrobenzol war quantitativ umgewandelt. Im Filtrat befanden sich 19,9 g Methyl-N-Phenylcarbamat und 4,11 g Anilin.

Beispiel 12

20,8 g Nitrobenzol, 2 g Schwefel, 3,21 g Anilin, 2,36 g Kaliumacetat, 3 g 1,4-Benzochinon, 0,87 g Morpholin und 169 g abs. Äthanol wurden, wie in Beispiel 8 beschrieben, umgesetzt. Das Nitrobenzol war zu 96,2% umgewandelt. Im Filtrat befanden sich 18,7 g Äthyl-N-phenylcarbamat und 3,1 g Anilin.

Beispiel 13

Beispiel 3 wurde mit 2,1 g 1,8-Diazabicyclo[5. 4. 0]-undecen-7 anstatt Kaliumacetat wiederholt. Das Nitrobenzol war quantitativ umgewandelt. Im Filtrat befanden sich 21,3 g Äthyl-N-phenylcarbamat und 2,8 g Anilin.

Beispiel 14

Beispiel 8 wurde mit 1 g Schwefel anstatt 2 g Schwefel wiederholt. Das Nitrobenzol war quantitativ umgewandelt. Im Filtrat befanden sich 19,7 g Äthyl-N-phenylcarbamat und 5,0 g Anilin.

Vergleichsbeispiel 14 a

Beispiel 14 wurde ohne Morpholin wiederholt. Das Nitrobenzol war zu 39,1% umgesetzt. Im Filtrat befanden sich 5,5 g Äthyl-N-phenylcarbamat und 2,7 g Anilin.

Beispiel 15

Beispiel 14 wurde mit 1,9 g Kaliumcyanat anstatt Kaliumrhodanid wiederholt. Das Nitrobenzol war quantitativ umgewandelt. Im Filtrat befanden sich 20,7 g Äthyl-N-phenylcarbamat und 3,2 g Anilin.

Beispiel 16

Beispiel 4 wurde mit 4 g N,N,N', N'-Tetramethyl-thiuramdisulfid anstatt Schwefel wiederholt. Das Nitrobenzol war quantitativ umgewandelt. Im Filtrat befanden sich 18,6 g Äthyl-N-phenylcarbamat und 2,9 g Anilin.

Beispiel 17

Beispiel 8 wurde unter Veränderung der Gewichtsverhältnisse Eisen-III-oxid zu Vanadinpentoxid wie 1:1 wiederholt. Das Nitrobenzol war quantitativ umgewandelt. Im Filtrat befanden sich 18,1 g Äthyl-N-phenylurethan und 7,0 g Anilin.

Beispiel 18

Beispiel 11 wurde mit 0,64 g Di-n-butylamin anstatt Morpholin wiederholt. Das Nitrobenzol war quantitativ umgewandelt. Im Filtrat befanden sich 20,8 g Methyl-N-phenylcarbamat und 4,0 g Anilin.

Beispiel 19

26,6 g 4-Nitrochlorbenzol, 2,0 g Schwefel, 0,2 g metallisches Selen, 4,4 g 4-Chloranilin, 2,36 g Kaliumacetat, 3 g Metalloxidgemisch gemäss Beispiel 1, 1,28 g Di-n-butylamin und 169 g abs. Äthanol wurden nach Beispiel 1 bei 180°C zur Reaktion gebracht. Die Aufarbeitung dieses Ansatzes entsprechend Beispiel 1 erbrachte 24,5 g Äthyl-N-(4-chlorphenyl)-carbamat,Kp.:118–120°C/ 0,27 mbar. Die Substanz war nach gaschromatographischer Analyse zu 98,5% rein.

Beispiel 20

25,46 g 2,4-Dinitrotoluol, 1,96 g Kaliumacetat, 0,2 g metallisches Selen, 3,5 g 2,4-Diaminotoluol, 2,5 g Metalloxidgemisch gemäss Beispiel 1, 0,64 g Di-n-butylamin und 140 g abs. Äthanol wurden in einen 0,7 l Autoklaven eingebracht. Die Luft im Autoklaven wurde durch Stickstoffgas und dann durch Kohlenoxid ersetzt. Anschliessend wurde Kohlenoxid unter Druck in den Autoklaven geleitet bis der Anfangsdruck von 100 bar bei Raumtemperatur erreicht war. Das Reaktionssystem wurde unter Rühren aufgeheizt und 1 Stunde auf 180°C gehalten. Die nach der Flüssigkeitschromatographie vorgenommene Analyse des vom Selen und Metalloxidgemisch abgetrennten Filtrats ergab eine quantitative Umsetzung des 2,4-Dinitrotoluol. Das Filtrat enthielt 29,5 g 2,4-Diäthoxycarbonylaminotoluol.

Vergleichsbeispiel 20 a

Beispiel 20 wurde ohne Di-n-butylamin wiederholt. Das Filtrat enthielt 22,9 g 2,4-Diäthoxycarbonylaminotoluol.

Beispiel 21

Beispiel 20 wurde unter Zusatz von 1 g Schwefel und mit 140 g Methanol anstatt Äthanol wiederholt. Das Reaktionsgemisch wurde von unlöslichen Bestandteilen heiss abfiltriert und das Filtrat langsam auf -10°C abgekühlt. Man filtrierte den gebildeten Kristallbrei ab, wusch zweimal mit 15 ml kaltem Methanol nach und erhielt nach Trocknung 20,5 g Dimethoxycarbonylaminotoluol vom Fp.: 167–169°C.

Vergleichsbeispiel 21 a

Beispiel 21 wurde ohne Di-n-butylamin wiederholt. Nach entsprechender Aufarbeitung erhielt

man 14,8 g Dimethoxycarbonylaminotoluol vom Fp.: 165–167°C. Die IR-Aufnahmen der isolierten Verbindungen aus Beispiel 21 und Vergleichsbeispiel 21 a waren identisch.

Beispiel 22

40,0 g 2,4-Dinitrotoluol, 2,0 g Kaliumacetat, 0,2 g metallisches Selen, 1,0 g Schwefel, 4,0 g 2,4-Diaminotoluol, 4,0 g Metalloxidgemisch gemäss Beispiel 1, 1,28 g Di-n-butylamin und 350 g abs. Äthanol wurden in einen 1,3 l Autoklaven eingebracht und entsprechend Beispiel 20 2 Stunden bei 160°C zur Reaktion gebracht. Das 2,4-Dinitrotoluol war quantitativ umgesetzt. Das Filtrat enthielt 43,2 g 2,4-Diäthoxycarbonylaminotoluol.

Beispiel 23

Beispiel 20 wurde unter Zusatz von 1,0 g Schwefel bei 170°C wiederholt. Das 2,4-Dinitrotoluol war quantitativ umgesetzt. Das Filtrat enthielt 31,0 g 2,4-Diäthoxycarbonylaminotoluol.

Vergleichsbeispiel 23 a

Beispiel 23 wurde ohne Di-n-butylamin wiederholt. Das Filtrat enthielt 20,2 g 2,4-Diäthoxycarbonylaminotoluol.

Beispiel 24

20,8 g Nitrobenzol, 0,1 g metallisches Selen, 3,21 g Anilin, 2,36 g Kaliumacetat, 3 g eines Metalloxidgemisches aus Eisen-III-oxid und Vanadinpentoxid im Gewichtsverhältnis 11:1, 0,64 g Di-n-butylamin und 169 g abs. Äthanol wurden in einen 0,7 l Autoklaven eingebracht. Der Autoklav wurde mit Stickstoff und nachfolgend mit Kohlenmonoxid gespült. Anschliessend wurde Kohlenmonoxid in den Autoklaven unter Druck eingeleitet, bis der Ausgangsdruck 100 bar erreichte. Unter Rühren wurde auf 170°C aufgeheizt und 1 Stunde bei 170°C nachgerührt. Die gaschromatographische Analyse ergab eine quantitative Umwandlung des Nitrobenzols. Im Filtrat befanden sich 26,9 g Äthyl-N-phenylcarbamat und 3,2 g Anilin.

Vergleichsbeispiele 24 a

Beispiel 24 wurde ohne Di-n-butylamin wiederholt. Im Filtrat befanden sich 23,8 g Äthyl-N-phenylcarbamat und 3,5 g Anilin.

Beispiel 25

51 g Nitrobenzol, 0,5 g Selen, 12 g Anilin, 4 g Kaliumacetat, 4 g eines Metalloxidgemisches gemäss Beispiel 1, 1,28 g Di-n-butylamin und 300 g abs. Äthanol wurden in einen 1,3 l Autoklaven eingebracht. Der Autoklav wurde mit Stickstoff und nachfolgend mit Kohlenmonoxid gespült. Anschliessend wurde Kohlenmonoxid in den Autoklaven unter Druck eingeleitet, bis der Ausgangsdruck 120 bar erreichte. Unter Rühren wurde auf 150 °C aufgeheizt und 2 Stunden bei 150°C nachgerührt. Die gaschromatographische Analyse ergab eine quantitativen Umwandlung des Nitrobenzols. Im Filtrat befanden sich 77,2 g Äthyl-N-phenylcarbamat und 5,1 g Anilin.

Beispiel 26

42 g Nitrobenzol, 4 g Schwefel, 12 g Anilin, 2 g Kaliumacetat, 4 g eines Metalloxidgemischs aus Eisen-III-oxid und Vanadinpentoxid im Gewichtsverhältnis 11:1, 1,28 g Di-n-butylamin und 300 g abs. Methanol wurden, wie in Beispiel 25 beschrieben 2 Stunden bei 160°C umgesetzt. Das Nitrobenzol war quantitativ umgewandelt. Im Filtrat befanden sich 45,9 g Methyl-N-phenylcarbamat und 11,7 g Anilin.

**Patentansprüche**

1. Verfahren zur Herstellung von Urethanen durch Umsetzung von aromatischen Nitroverbindungen mit aliphatischen, cycloaliphatischen oder araliphatischen Alkoholen und Kohlenmonoxid in Gegenwart von

a) Schwefel und/oder Selen und/oder Verbindungen dieser Elemente, sowie

b) aromatische Aminoverbindungen und/oder aromatische Harnstoffverbindungen, sowie

c) tertiäre organische Amine und/oder Alkalisalze schwacher Säuren aufweisenden Katalysatorensystemen, sowie

d) Oxidationsmittel ausgewählt aus der Gruppe bestehend aus Sauerstoff, oxidierend wirkenden, chemisch gebundenen Sauerstoff enthaltenden organischen Verbindungen und oxidierend wirkenden, chemisch gebundenen Sauerstoff enthaltenden anorganischen Verbindungen von Metallen der 1., 2., 5.–8. Nebengruppe des Periodensystems der Elemente,

dadurch gekennzeichnet, dass man Katalysatorensysteme verwendet, welche als zusätzliche Komponente

e) Ammoniak und/oder aliphatische, araliphatische, cycloaliphatische oder heterocyclische Amine mit mindestens einem an Aminstickstoff gebundenen Wasserstoffatom enthalten.

2. Verfahren gemäss Anspruch 1, dadurch gekennzeichnet, dass man als Komponente e) sekundäre Amine verwendet.

3. Verfahren gemäss Anspruch 1 und 2, dadurch gekennzeichnet, dass man als Komponente c) Diazabicyclo [2. 2. 2] octan verwendet.

4. Verfahren gemäss Anspruch 1 bis 3, dadurch gekennzeichnet, dass man als Komponente c) ein tert. Amin in Kombination mit einem Salz der Formel

MeX

eingesetzt wird, wobei

Me für ein Alkalimetall-Kation und

X für ein Jodid-, Cyanat- oder Rhodanid-Anion steht.

5. Verfahren gemäss Anspruch 1 bis 4, dadurch gekennzeichnet, dass man als aromatische Nitroverbindung Nitrobenzol verwendet.

6. Verfahren gemäss Anspruch 1 bis 4, dadurch gekennzeichnet, dass man als aromatische Nitroverbindung 2,4- oder 2,6-Dinitroltoluol oder deren Gemische verwendet.

7. Verfahren gemäss Anspruch 1 bis 6, dadurch

gekennzeichnet, dass man als Alkohol Äthylalkohol oder Methylalkohol verwendet.

**Patent Claims**

1. A process for the preparation of urethanes by the reaction of aromatic nitro compounds with aliphatic, cycloaliphatic or araliphatic alcohols and carbon monoxide in the presence of:
a) sulphur and/or selenium and/or compounds of these elements and
b) aromatic amino compounds and/or aromatic urea compounds and
c) catalyst systems containing tertiary organic amines and/or alkali metal salts of weak acids, and
d) oxidizing agents selected from the group constisting of oxygen, oxidizing organic compounds containing chemically bound oxygen and oxidizing inorganic compounds of metals of the 1st, 2nd and 5th to 8th sub-Groups of the Periodic System of Elements containing chemically bound oxygen,
characterised in that catalyst systems are used which contain, as additional component
e) ammonia and/or aliphatic, araliphatic, cycloaliphatic or heterocyclic amines having at least one hydrogen atom bound to an amine nitrogen.
2. A process according to Claim 1, characterised in that secondary amines are used as component e).
3. A process according to Claims 1 and 2, characterised in that diazabicyclo [2,2,2]-octane is used as component c).
4. A process according to Claims 1 to 3, characterised in that a tertiary amine in combination with a salt of the formula

MeX

wherein
Me represents an alkali metal cation and
X represents an iodide, cyanate or thiocyanate anion, is used as component c).
5. A process according to Claims 1 to 4, characterised in that the aromatic nitro compound used is nitrobenzene.
6. A process according to Claims 1 to 4, characterised in that the aromatic nitro compound used is 2,4- or 2,6-dinitrotoluene or mixtures thereof.
7. A process according to Claims 1 to 6, characterised in that the alcohol used is ethyl alcohol or methyl alcohol.

**Revendications**

1. Procédé de production d'uréthannes par réaction de composés aromatiques nitrés avec des alcools aliphatiques, cycloaliphatiques ou araliphatiques et l'oxyde de carbone en présence
a) de soufre et/ou de sélénium et/ou de composés de ces éléments, ainsi que
b) de composés aminés aromatiques et/ou de composés aromatiques d'urée, ainsi que
c) de système catalyseurs comportant des amines organiques tertiaires et/ou des sels de métaux alcalins d'acides faibles, ainsi que
d) d'agents oxydants choisis dans le groupe comprenant l'oxygène, des composés organiques à action oxydante, renfermant de l'oxygène lié chimiquement et des composés inorganiques à action oxydante, renfermant de l'oxygène lié chimiquement, de métaux des sous-groupes 1, 2 et 5-8 de la Classification Périodique des Eléments, caractérisé en ce qu'on utilise des systèmes catalyseurs qui contiennent comme autre composant
e) de l'ammoniac et/ou des amines aliphatiques, araliphatiques, cycloaliphatiques ou hétérocycliques ayant au moins un atome d'hydrogène lié à l'azote d'amine.
2. Procédé suivant la revendication 1, caractérisé en ce qu'on utilise des amines secondaires comme composant e).
3. Procédé suivant l'une des revendications 1 et 2, caractérisé en ce qu'on utilise le diazabicyclo-[2.2.2]-octane comme composant c).
4. Procédé suivant l'une des revendications 1 à 3, caractérisé en ce qu'on utilise comme composant c) une amine tertiaire en association avec un sel de formule:

MeX

dans laquelle:

Me est un cation de métal alcalin et
X est un union iodure, cyanate ou sulfocyanure.
5. Procédé suivant l'une des revendications 1 à 4, caractérisé en ce qu'on utilise le nitrobenzène comme composé aromatique nitré.
6. Procédé suivant l'une des revendications 1 à 4, caractérisé en ce qu'on utilise le 2,4- ou le 2,6-dinitrotoluène ou leurs mélanges comme composé aromatique nitré.
7. Procédé suivant l'une des revendications 1 à 6, caractérisé en ce qu'on utilise comme alcool l'alcool éthylique ou l'alcool méthylique.